# EUROPEAN PATENT APPLICATION

(11) **EP 1 935 450 A1**
(43) Date of publication of application: **25.06.2008**
(21) Application number: 07023020.6
(22) Date of filing: 28.11.2007
(51) Int. Cl.: A61N 1/37

(54) **Implantable medical device comprising magnetic field detector**

(30) Priority: 21.12.2006 US 614333
(71) Applicant: BIOTRONIK CRM Patent AG, 6341 Baar (CH)
(72) Inventor: Digby, Dennis, Wilsonville Oregon 97070 (US); Wiggins, David, Tualatin OR 97062-7123 (US); Berthelsdorf, Richard, Dr., Newberg, OR 97132 (US)
(74) Representative: Lindner-Vogt, Karin L.

(57) **Abstract**

An implantable medical device comprises an electronic control unit and a magnetic resonance imaging magnetic field detector that is connected to said control unit. The magnetic resonance imaging magnetic field detector is adapted to generate a signal being characteristic for a magnetic field as used for magnetic resonance imaging (MRI) and the control unit is adapted to positively recognize a presence of a magnetic field as used for magnetic resonance imaging (MRI) and to cause the implantable medical device to enter an MRI-safe mode of operation.

## Description

The invention refers to an implantable medical device such as an implantable pacemaker or an implantable cardioverter/defibrillator (ICD).

For more than 35 years, manufacturers have used a reed switch in cardiac implants to set the implant into a mode of operation that is commonly referred to as the magnet mode. The reed switch is normally open, and is closed when a permanent magnet is brought into close proximity to the implant.

MRI is a diagnostic tool that has become increasingly popular, and is typically contraindicated for pacemaker and ICD patients due to possible mechanical forces, heating of leads, permanent damage to the electronic circuit or inappropriate therapy (e.g. loss of sensing or pacing at the UTR). An MRI has a static magnetic field that typically has a strength of 1.5T, and newer MRI machines with fields of 3T or higher are becoming available. When it is exposed to such a field, a reed switch will remain closed, placing the device into its magnet mode. However, a reed switch is unable to differentiate between a magnetic field of 2mT or 1.5T.

The use of a Hall sensor to detect magnetic fields is a known alternative to reed switches. The Hall effect sensor has the advantage that its output voltage is proportional to the strength of the magnetic field, enabling determination of whether the magnetic field is from a normal permanent magnet or an MRI. US 6,937,906 discloses an implantable pacemaker being able to detect the MRI magnetic field, as well as automatically changing the mode of the device to "a second sensing mode less effected by the MRI interference signal".

Another known alternative to a reed switch is a GMR sensor, see US 6,101,417.

It is an object of the invention to provide an implantable medical device that has a sensor that is able to differentiate between low-level and very high-level magnetic fields, as that would make it possible to automatically place the device into a patient safe "MRI mode" if the presence of an MRI magnetic field were detected.

This object is achieved by an implantable medical device comprising an electronic control unit and an magnetic resonance imaging magnetic field detector that is connected to said control unit, wherein the magnetic resonance imaging magnetic field detector is adapted to generate a signal being characteristic for a magnetic field as used for magnetic resonance imaging (MRI) and wherein the control unit is adapted to positively recognize a presence of a magnetic field as used for magnetic resonance imaging (MRI) and to cause the implantable medical device to enter an MRI-safe mode of operation.

The magnetic resonance imaging magnetic field detector may comprise a bandpass receiver comprising an antenna and a receiver output that is connected to a comparator to compare a bandpass receiver output signal to a threshold value, said threshold value being adapted to a minimum output value to be expected in an MRI environment and wherein said control unit is adapted to cause the implantable medical device to enter an MRI-safe mode of operation upon reception of a comparator output signal indicating a receiver output signal exceeding said threshold value.

Alternatively or additionally the magnetic resonance imaging magnetic field detector may comprise a giant magnetoresistive ratio sensor that is adapted and arranged to put out a sensor signal that depends on both, magnitude and direction of a magnetic field, and wherein the control unit is adapted to respond to an output signal from said giant magnetoresistive ratio sensor differently depending on whether the sensor output signal represents
a: no or a very low magnetic field up to an order of 0,1 mT or
b: a medium magnetic field in the order of 1 mT or
c: a strong magnetic field in the order of 1 T or more,

The control unit is further adapted to cause the implantable medical device to enter said MRI-safe mode of operation when the sensor output signal represents a strong magnetic field in the order of 1 T or more.

With respect to an implantable medical device comprising a bandpass receiver the antenna of said bandpass receiver is preferably a programming coil used for programming the implantable medical device.

With respect to a MRI safe mode of operation it is preferred that the control unit is adapted to cause the implantable medical device to enter a V00 mode of operation upon reception of a comparator output signal indicating a receiver output signal exceeding said threshold value.

Alternatively, the control unit may be adapted to cause the implantable medical device to enter a D00 mode of operation upon reception of a comparator output signal indicating a receiver output signal exceeding said threshold value.

In yet another alternatively preferred embodiment the control unit is adapted to cause a system reset of the implantable medical device upon reception of a comparator output signal indicating a receiver output signal exceeding said threshold value.

Preferably the GMR sensor is adapted to recognize a magnetic field vector depending on magnitude and direction of a magnetic field and to compensate said magnetic field vector for B0- and G-fields.

Preferably the GMR sensor has a high sensitivity to detect magnetic fields in the range of 1 mT to 100mT, in order to be able to detect the presence of the magnets in a programmer head or any bar or donut magnet that may be used by a patient or clinic to put the device into its magnet mode. With appropriate signal conditioning circuits it is possible to detect the presence of very high magnetic fields of an MRI diagnostic test. MRI magnetic fields are typically 1.5T or higher.

Preferably, the MRI magnetic field detector is adapted to use averaging techniques to self-calibrate the GMR sensor.

The above and other aspects, features and advantages of the present invention will be more apparent from the following more particular description thereof, presented in conjunction with the following drawings wherein:
- FIG. 1: shows a dual chamber pacemaker connected to leads placed in a heart.
- FIG. 2: is a block diagram of a heart stimulator according to the invention.
- FIG. 3: is a GMR Equivalent circuit.
- FIG. 4: shows typical characteristics of a GMR sensor for a normal magnetic field strength range
- FIG. 5: shows typical characteristics of a GMR sensor for an extended magnetic field strength range
- FIG. 6: shows typical characteristics of the GMR Sensors with a Constant Current Supply GMR Supply Voltage and Output over an Extended Field Strength Range
- FIG. 7: shows an embodiment of the invention featuring a single GMR resistor in series with a conventional low inductance resistor
- FIG. 8: shows an embodiment of the invention featuring two GMR sensors that are mounted onto the electronic circuit at 90 degrees with respect to each other
- FIG. 9: shows an embodiment of the invention using a full-bridge GMR sensor.
- FIG. 10: a receiver circuit of an alternative embodiment of the invention featuring a receiver that is adapted
- FIG. 11: shows a dual antenna design for the embodiment according to FIG. 10.

The following description is of the best mode presently contemplated for carrying out the invention. This description is not to be taken in a limiting sense, but is made merely for the purpose of describing the general principles of the invention. The scope of the invention should be determined with reference to the claims.

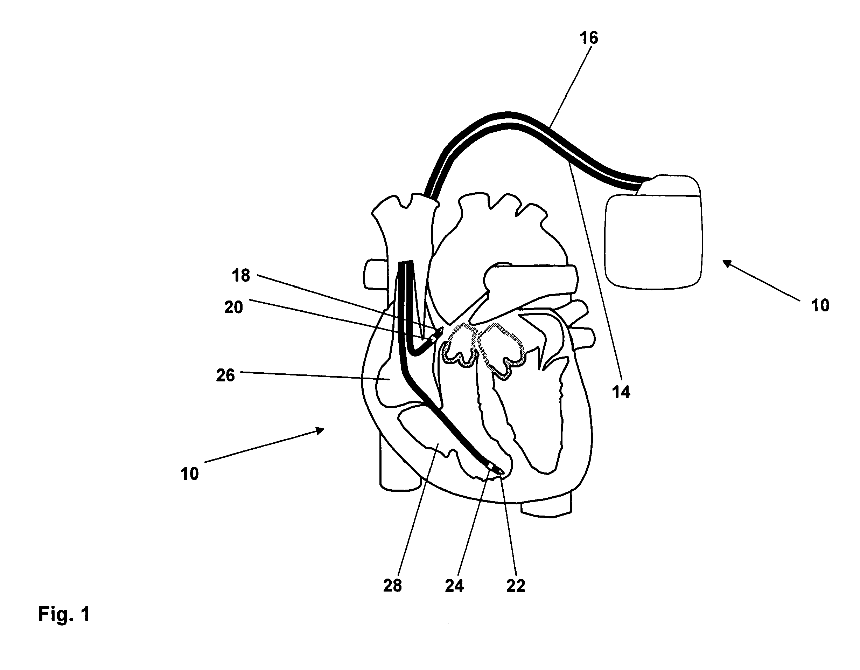

In FIG. 1 a dual chamber pacemaker 10 as heart stimulator connected to pacing/sensing leads placed in a heart 12 is illustrated. The pacemaker 10 is electrically coupled to heart 12 by way of leads 14 and 16. Lead 14 has a pair of right atrial electrodes 18 and 20 that are in contact with the right atria 26 of the heart 12. Lead 16 has a pair of electrodes 22 and 24 that are in contact with the right ventricle 28 of heart 12. Electrodes 18 and 22 are tip- electrodes at the very distal end of leads 14 and 16, respectively. Electrode 18 is a right atrial tip electrode RA-Tip and electrode 22 is a right ventricular tip electrode 22. Electrodes 20 and 24 are ring electrodes in close proximity but electrically isolated from the respective tip electrodes 18 and 22. Electrode 20 forms a right atrial ring electrode RARing and electrode 24 forms a right ventricular ring electrode RV-Ring.

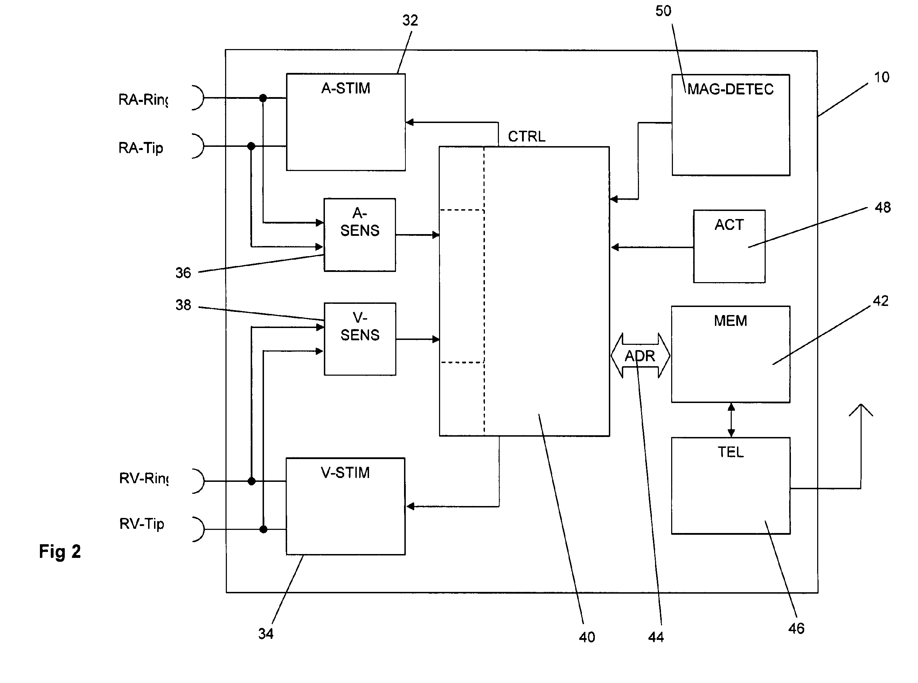

Referring to FIG. 2 a simplified block diagram of a dual chamber pacemaker 10 is illustrated. During operation of the pacemaker leads 14 and 16 are connected to respective output/input terminals of pacemaker 10 as indicated in Fig. 1 and carry stimulating pulses to the tip electrodes 18 and 22 from an atrial stimulation pulse generator A-STIM 32 and a ventricular pulse generator V-STIM 34, respectively. Further, electrical signals from the atrium are carried from the electrode pair 18 and 20, through the lead 14, to the input terminal of an atrial channel sensing stage A-SENS 36; and electrical signals from the ventricles are carried from the electrode pair 22 and 24, through the lead 16, to the input terminal of a ventricular sensing stage V-SENS 38.

Controlling the dual chamber pacer 10 is a control unit CTRL 40 that is connected to sensing stages A-SENS 36 and V-SENS 38 and to stimulation pulse generators A-STIM 32 and V-STIM 34. Control unit CTRL 40 receives the output signals from the atrial sensing stage A-SENS 36 and from the ventricular sensing stage V-SENS 38. The output signals of sensing stages A-SENS 36 and V-SENS 38 are generated each time that a P-wave representing an intrinsic atrial event or an R-wave representing an intrinsic ventricular event, respectively, is sensed within the heart 12. An As-signal is generated, when the atrial sensing stage A-SENS 36 detects a P-wave and a Vs-signal is generated, when the ventricular sensing stage V-SENS 38 detects an R-wave.

Control unit CTRL 40 also generates trigger signals that are sent to the atrial stimulation pulse generator A-STIM 32 and the ventricular stimulation pulse generator V-STIM 34, respectively. These trigger signals are generated each time that a stimulation pulse is to be generated by the respective pulse generator A-STIM 32 or V-STIM 34. The atrial trigger signal is referred to simply as the "A-pulse", and the ventricular trigger signal is referred to as the "V-pulse". During the time that either an atrial stimulation pulse or ventricular stimulation pulse is being delivered to the heart, the corresponding sensing stage, A-SENS 36 and/or V-SENS 38, is typically disabled by way of a blanking signal presented to these amplifiers from the control unit CTRL 40, respectively. This blanking action prevents the sensing stages A-SENS 36 and V-SENS 38 from becoming saturated from the relatively large stimulation pulses that are present at their input terminals during this time. This blanking action also helps prevent residual electrical signals present in the muscle tissue as a result of the pacer stimulation from being interpreted as P-waves or R-waves.

Furthermore, atrial sense events As recorded shortly after delivery of a ventricular stimulation pulses during a preset time interval called post ventricular atrial refractory period (PVARP) are generally recorded as atrial refractory sense event Ars but ignored.

Control unit CTRL 40 comprises circuitry for timing ventricular and/or atrial stimulation pulses according to an adequate stimulation rate that can be adapted to a patient's hemodynamic need as pointed out below.

Still referring to FIG. 2, the pacer 10 includes a memory circuit MEM 42 that is coupled to the control unit CTRL 40 over a suitable data/address bus ADR 44. This memory circuit MEM 42 allows certain control parameters, used by the control unit CTRL 40 in controlling the operation of the pacemaker 10, to be programmably stored and modified, as required, in order to customize the pacemaker's operation to suit the needs of a particular patient. Such data includes the basic timing intervals used during operation of the pacemaker 10.

Further, data sensed during the operation of the pacemaker may be stored in the memory MEM 42 for later retrieval and analysis. This includes atrioventricular interval data that are acquired by the control unit CTRL 40. control unit CTRL 40 is adapted to determine the atrioventricular interval data as required for automatic atrioventricular interval analysis by determining the time interval between an atrial event, either sensed (As) or stimulated (Ap) and an immediately following ventricular sensed event Vs as indicated by the ventricular sensing stage V-SENS 38.

A telemetry circuit TEL 46 is further included in the pacemaker 10. This telemetry circuit TEL 46 is connected to the control unit CTRL 40 by way of a suitable command/data bus. Telemetry circuit TEL 46 allows for wireless data exchange between the pacemaker 10 and some remote programming or analyzing device which can be part of a centralized service center serving multiple pacemakers.

The pacemaker 10 in FIG. 1 is referred to as a dual chamber pacemaker because it interfaces with both the right atrium 26 and the right ventricle 28 of the heart 12. Those portions of the pacemaker 10 that interface with the right atrium, e.g., the lead 14, the P-wave sensing stage A-SENSE 36, the atrial stimulation pulse generator A-STIM 32 and corresponding portions of the control unit CTRL 40, are commonly referred to as the atrial channel. Similarly, those portions of the pacemaker 10 that interface with the right ventricle 28, e.g., the lead 16, the R-wave sensing stage V-SENSE 38, the ventricular stimulation pulse generator V-STIM 34, and corresponding portions of the control unit CTRL 40, are commonly referred to as the ventricular channel.

In order to allow rate adaptive pacing in a DDDR or a DDIR mode, the pacemaker 10 further includes a physiological sensor ACT 48 that is connected to the control unit CTRL 40 of the pacemaker 10. While this sensor ACT 48 is illustrated in FIG. 2 as being included within the pacemaker 10, it is to be understood that the sensor may also be external to the pacemaker 10, yet still be implanted within or carried by the patient.

Control unit CTRL 40 is adapted to put the pacemaker 10 into a VVO or a DDO mode of operation wherein either only the ventricle or the ventricle and the atrium are stimulated with fixed stimulation rate and no sensing nor any inhibition is provided. For such a mode of operation the ventricular stimulation pulse generator V-STIM 32 or both, the ventricular stimulation pulse generator V-STIM 32 and the atrial stimulation pulse generator 34 can be connected to a fixed rate oscillator that is insensitive to MRI magnetic fields.

A further feature of pacemaker 10 is a magnetic field detector MAG-SENS 50 that is connected to control unit CTRL 40. The magnetic field detector MAG-SENS 50 is adapted to generate a detect signal being characteristic for a magnetic field as used for magnetic resonance imaging (MRI). The control unit CTRL 40 is adapted to process the detect signal generated by the magnetic field detector MAG-DETEC 50 and to thus positively recognize a presence of a magnetic field as used for magnetic resonance imaging (MRI). The control unit CTRL 40 responds to detection of a presence of a magnetic field as used for magnetic resonance imaging by causing the implantable medical device to enter an MRI-safe mode of operation.

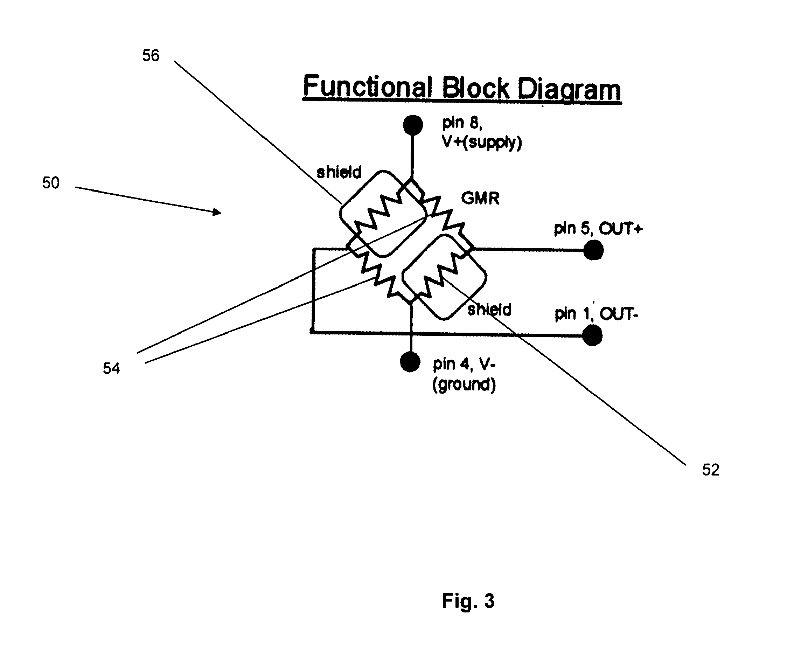

In one embodiment, the magnetic field detector MAG-DETEC 50 is a giant magnetoresistive (GMR) sensor as depicted in Figures 3, 7, 8 and 9.

When a magnetic field is applied, the GMR effect results in a decrease in the electrical resistance of a multilayer structure comprised of alternating layers of ferromagnetic and paramagnetic thin films. The GMR sensor typically comprises 4 equal value resistors, fabricated using thin-film technology on a silicon substrate, in a Wheatstone Bridge configuration. However, it is also possible to fabricate a GMR sensor with one or more resistors.

A typical implementation of a GMR sensor is shown in FIG. 3. 4 resistors 52 and 54 are arranged in a Wheatstone Bridge configuration. The 4 resistors are fabricated using the same materials and with the same resistance value. Two resistors 52 have magnetic shields 56 placed over them; the other two resistors 54 are unshielded and may have flux concentrators to concentrate the magnetic field into them. The resistors are typically within the range of 2 k□ to 50 k□. Due to these low resistance values, special techniques are required to reduce the average power required by the GMR control and processing circuit for use in an implantable medical device. Reduction of the average power to a level acceptable for an implanted device may be done by using a low operating voltage or "strobing" the GMR sensor with a low duty cycle.

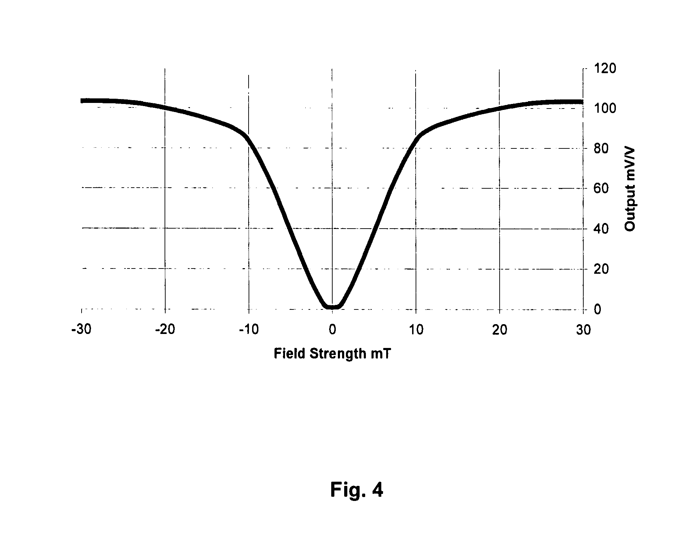

The behaviour of the GMR sensor of FIG. 3 depends on the strength of a magnetic field to which the GMR sensor is exposed. With no magnetic field applied, the output of the Wheatstone Bridge (between OUT+ and OUT-) will be close to 0mV, although there may be a small offset due to the earth's magnetic field and/or resistor value mismatch. When a magnetic field is applied to the GMR sensor in the axis of sensitivity, the unshielded resistors will decrease in resistance as the magnetic field strength increases. The shielded resistors also change in resistance, but by only an insignificant amount compared to the change in the unshielded resistors over the operating range of the GMR sensor. Referring to FIG. 3 this results in an increase in the voltage difference between OUT- and OUT+ when a voltage is applied between V- and V+. The change in resistance is independent of whether the field is N-S or S-N. The change in GMR resistance from that due to the background earth's magnetic field (approx. 0.05 mT) to magnetic saturation of the device results in a typical resistance change of 12% to 16%. FIG. 4 shows the typical output characteristics of a GMR when a magnetic field is applied in both directions. GMR sensors can be manufactured with a wide range of sensitivities and magnetic field operating ranges.

The GMR sensor is sensitive to the axis of the magnetic field, as is also the case with a reed switch. The "window" where the orientation of the applied magnet axis results in the reed switch opening is similar to that of a GMR sensor in a practical application.

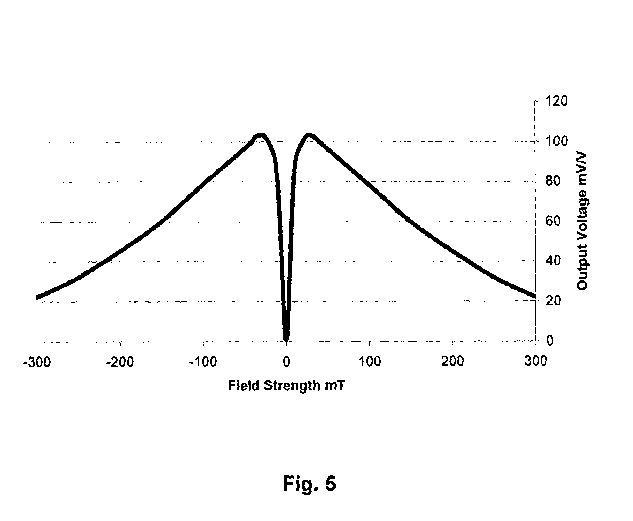

FIG. 5 shows the typical output characteristics of a GMR sensor when a large magnetic field above the saturation magnetic field is applied in both directions. As the magnetic field applied to a GMR sensor increases beyond the saturation level, the magnetic shields become ineffective, so that the shielded resistors begin decreasing in resistance at a higher rate than the unshielded resistors. The net effect is a tendency for the output of the GMR to reduce as the Wheatstone bridge balances, so that the output at very high magnetic fields might be interpreted by a simple GMR sensor's detection circuit as not meeting the magnet mode threshold. Such undesirable interpretation might occur if the device is exposed to the high magnetic field of an MRI machine.

The high static magnetic field of an MRI may cause mechanical forces to be applied to the implantable device if it contains any ferrous materials. This static magnetic field will close a convention reed switch and place the device in its magnet mode.

In addition to a very high static magnetic field, the MRI also has three gradient coils (one for each axis) which produce a high-level AC magnetic field and a pulsed RF signal. These EMI sources may cause a pacemaker or ICD to become inhibited or provide inappropriate therapy - for example, an ICD may inappropriately detect a "false" ventricular tachycardia and subsequently provide ATP pacing or shock therapy. In ICD's it is common for the "magnet mode" to disable the tachycardia or shock therapy, which is desirable during an MRI procedure.

These issues are addressed by the implantable medical device such a pacemaker 10 using a GMR sensor for providing a MRI magnetic field detector 50 for automatic MRI detection and reprogramming of the device to a patient safe mode

In one embodiment of pacemaker 10 a GMR sensor is used both for the detection of magnetic fields associated with a permanent magnet, as supplied to pacemaker and ICD patients and medical personnel or found in a device programming wand, and also for the detection of the very high magnetic field present during an MRI procedure. The outputs of the GMR sensor may be processed by control unit CTRL 40 to distinguish the two cases, so that pacemaker 10 can respond to them in different manners.

When the very high magnetic field of an MRI is detected, this information may be used by control unit CTRL 40 to automatically reprogram the device to a patient safe MRI safe mode (if the device has not already been placed into such a mode prior to the MRI). One example of a MRI Safe mode is VOO or DOO overdrive stimulation at a stimulation 20% above the programmed base pacing rate or the intrinsic rate, whichever is the highest.

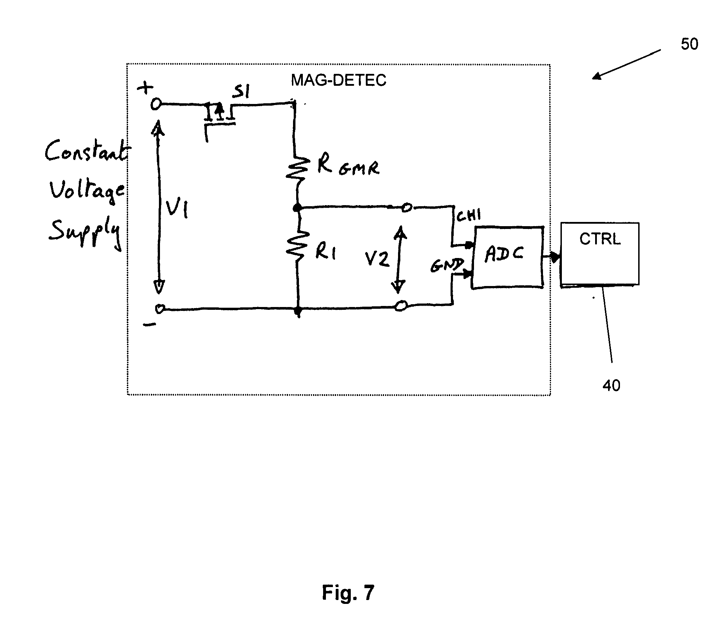

FIG. 7 shows one embodiment of a MRI magnetic field detector in combination with the control unit CTRL 40. A single GMR resistor R_{GMR} is placed in series with a conventional low inductance resistor R₁. The conventional resistor will remain stable when subjected to a magnetic field, while the GMR resistor will decrease in resistance as the applied magnetic field strength increases. Resistors R_{GMR} and R₁ form a potential divider network across a constant voltage supply V1 when the electronic switch S1 is closed. In Figures 7, 8 and 9, S1 is shown as a p-channel MOS transistor, although it could be constructed otherwise. To conserve power, switch S1 is used to apply voltage with a low duty cycle, for example 90µs every 0.5s. Voltage V2 is measured by an analog-to-digital converter ADC, or equivalently by an analog comparator, and is used by the control unit CTRL 40 to determine the relative magnitude of the magnetic field. It is desirable to be able to detect magnetic fields of 2mT and above in order to place the implanted device into its magnet mode. Calibration of the GMR circuit may be achieved by using a GMR with a well- defined sensitivity, and/or trimming R1, and/or programming a threshold value of the ADC output to set the magnet mode. The circuit can self-calibrate for the normal case of very small magnetic field (no magnet applied) by averaging the samples of V2. Since the application of a magnet occurs only occasionally and for a relatively short period of time, the long term average of V2 will be the result of the earth's magnetic field. The instantaneous value of V2, compared to the long term average, provides a measure of the applied magnetic field. In the presence of an MRI the resistance of R_{GMR} will be substantially reduced and thus may be detected by the control unit CTRL 40, allowing the device to be automatically reprogrammed to a Patient Safe MRI mode.

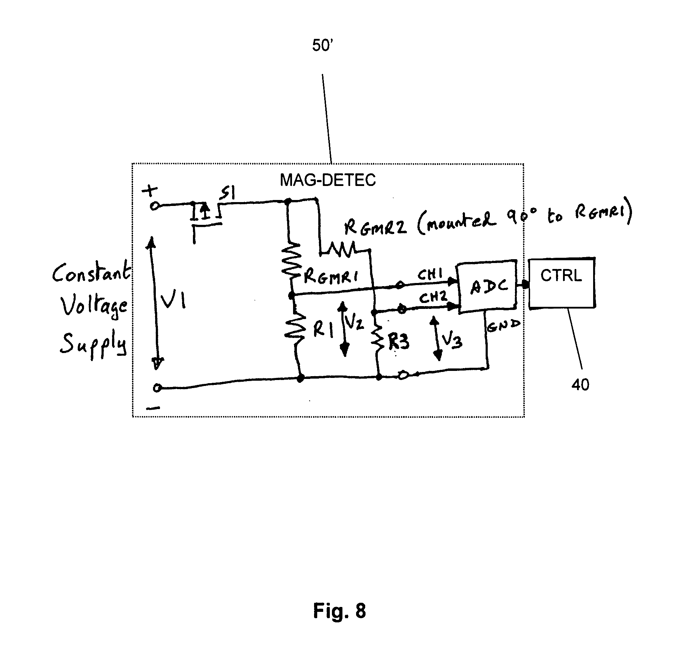

The GMR sensor is sensitive to the axis of the magnetic field, similar to the behavior of a reed switch. A magnetic field at right angles to the GMR axis of sensitivity will produce no output. To avoid this behavior, two GMR sensors may be mounted onto the electronic circuit at 90 degrees to each other to ensure that one or both GMR sensors are oriented in the axis of the magnetic field (this is impractical for reed switches due to their cost and size). FIG. 8 shows one method for this implementation, where the control unit CTRL 40 uses the lowest of the voltages at CH1 or CH2 to determine the presence of a magnetic field.

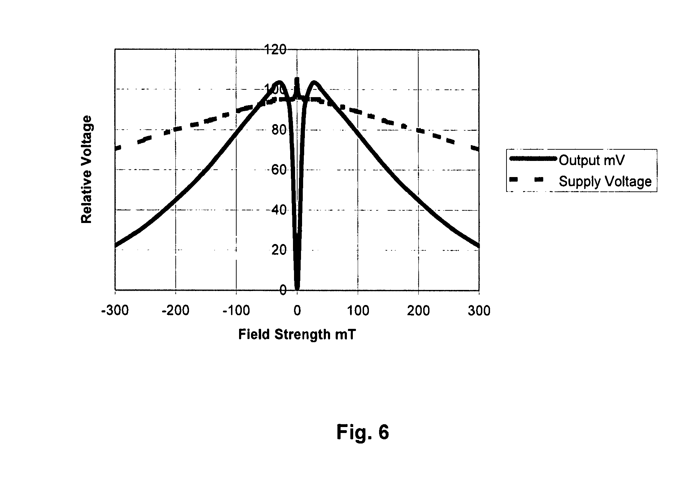

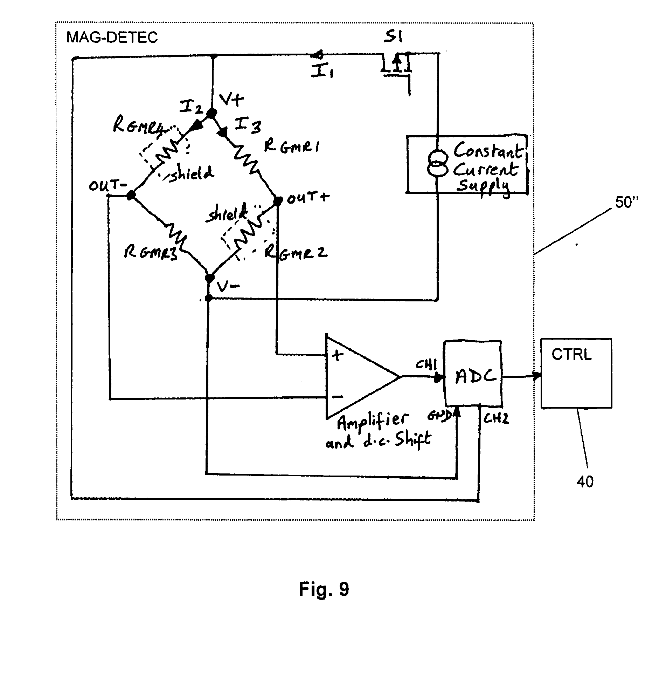

FIG. 9 shows a particularily preferred embodiment of the invention, using a full-bridge GMR sensor. One advantage of this approach is that it is more sensitive than the single GMR resistor approach described above. In addition, the full-bridge is inherently more stable with time and temperature, because any drift in the resistance values over time will be compensated automatically, since all resistors would be expected to change by the same amount. A constant current source I₁ is applied to the GMR sensor with a variable duty cycle controlled by switch S1. A typical duty cycle would close S1 for 91µs every 0.5s. The GMR sensor output voltage is measured between OUT+ and OUT- and the supply voltage is measured between V+ and V-. Both are sampled towards the end of the 91 us active interval to allow sufficient time for the measurement circuit to become stable. The characteristics of the two signals as a function of applied magnetic field are shown in FIG. 6. The GMR sensor output voltage is amplified, DC-shifted and applied to CH1 of the multiplexing ADC. The supply voltage may be connected directly to CH2 of the ADC, or to it via an amplifier or attenuator. The control unit CTRL 40 or other control circuit processes and averages the outputs from ADC channels CH1 and CH2 in order to determine the magnitude of the magnetic field.

The voltage between OUT+ and OUT- is used as a sensitive measurement for low magnetic field strengths only, while the voltage between V+ and V- is a crude measurement of magnetic field that is used to qualify the sensitive measurement. For the GMR sensor characteristics shown in FIG. 6, a decrease in the voltage between V+ and V- of 15% or more would indicate a magnetic field strength of >200mT. In this example, such an output signal would cause control unit CTRL 40 to place pacemaker 10 into its MRI safe mode. The high magnetic field strength detection limit may increase or decrease depending on the GMR sensor characteristics and the desired MRI safe mode threshold. Long term averaging of the signals at CH1 and CH2 can be used to self-calibrate the system.

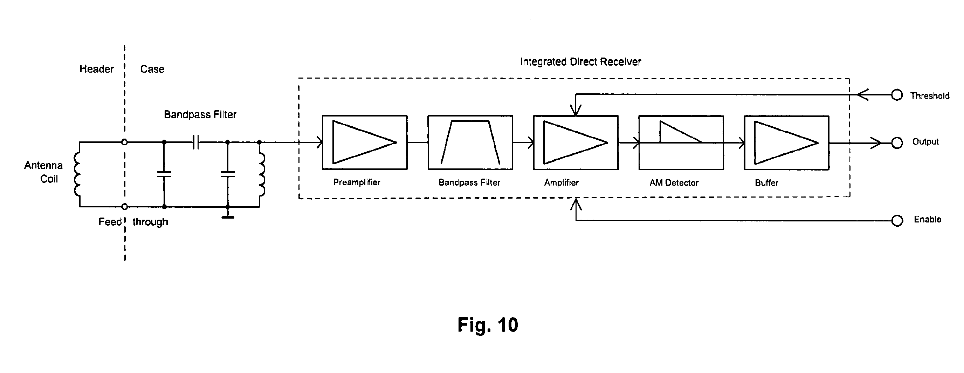

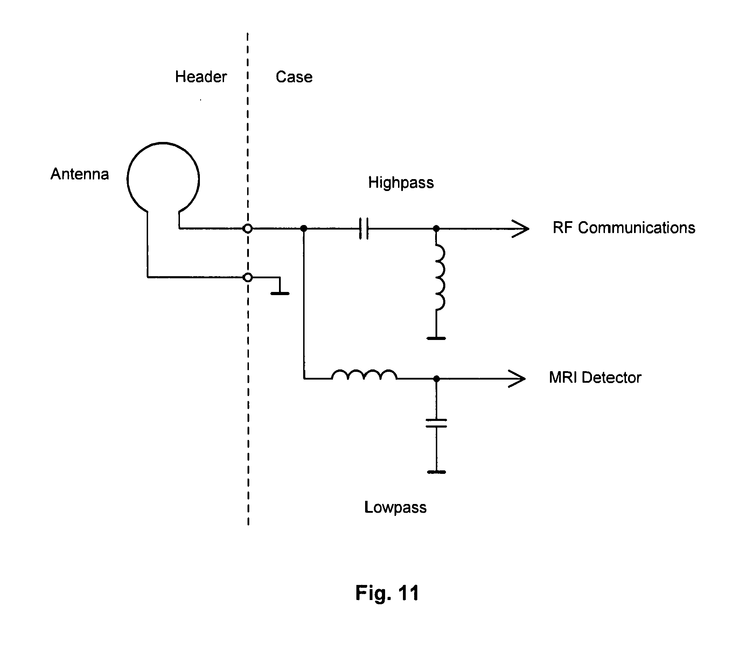

An alternative embodiment of the magnetic field detector MAG-DETEC 50 is shown in Figures 10 and 11.

The MRI magnetic field detector 50"" of Figures 10 and 11 is based on the principle that the signal to be detected is characterized by its strength, frequency range, duration, and repetition. In order to achieve sufficient specifity, the first two signal characteristics can be exploited with a bandpass receiver that is less sensitive than a normal communications receiver by a factor of about 100 in terms of antenna voltage, and thus will not erroneously detect background noise. The receiver output is a binary value indicating the current presence of a detected signal. The last two signal characteristics can be exploited by postprocessing the receiver output with hardwired or programmable digital circuitry that evaluates the pulse timing characteristics to further increase the specifity.

Exemplary embodiments of such MRI magnetic field detector 50"" are shown in Figures 10 and 11.

FIG. 10 shows a dedicated receiver.

As has been shown above, a small solenoid coil, located in the header of the implant, may be used as an antenna. If, e.g., the coil has a diameter of 2mm and 50 turns of 0.1mm wire with 0.1mm spacing, resulting in a length of 2.5mm, the induced peak voltage under above conditions is 370mV. This, together with the relatively low frequency, allows to use an ultra low-power direct receiver, without conversion to an intermediate frequency, according to the following block diagram (subsequent digital evaluation circuitry not shown).

The bandpass filters are designed for the expected range of excitation frequencies, e.g. 15MHz to 70MHz. The receiver may be a dedicted integrated circuit for flexible application. It may also be part of some other (e.g. CMOS) implant IC, and within that, components of an existing RF receiver may be reused.

The detection threshold is programmable to adapt to the actual signal path losses. The receiver may be operated continually in a pulsed manner with a low duty cycle to save power, or may be enabled by the noise detection circuitry of the implant, thus verifying the source of noise and triggering appropriate action, or it may be turned on by an external command.

FIG. 11 shows a shared antenna for MRI magnetic field detection.

In another embodiment, the dedicated antenna is replaced by an antenna, e.g., a loop antenna, that is already present for radio frequency communications purposes at higher frequencies, see FIG. 11. The advantage is that no additional feedthrough is required for a second antenna. The induced voltage may be lower due to a lower N·A product, requiring a somewhat more sensitive receiver, which could also be a superregenerative receiver. The dual use antenna is possible with a frequency-splitting diplexer circuitry as follows.

Although an exemplary embodiment of the present invention has been shown and described, it should be apparent to those of ordinary skill that a number of changes and modifications to the invention may be made without departing from the spirit and scope of the invention. This invention can readily be adapted to such devices by following the present teachings. All such changes, modifications and alterations should therefore be recognized as falling within the scope of the present invention.

## Claims

1. An implantable medical device comprising an electronic control unit and an magnetic resonance imaging magnetic field detector that is connected to said control unit, wherein the magnetic resonance imaging magnetic field detector is adapted to generate a signal being characteristic for a magnetic field as used for magnetic resonance imaging (MRI) and wherein the control unit is adapted to positively recognize a presence of a magnetic field as used for magnetic resonance imaging (MRI) and to cause the implantable medical device to enter an MRI-safe mode of operation.

2. The implantable medical device according to claim 1, wherein the magnetic resonance imaging magnetic field detector comprises a bandpass receiver comprising an antenna and a receiver output that is connected to a comparator to compare an bandpass receiver output signal to a threshold value, said threshold value being adapted to a minimum output value to be expected in an MRI environment and wherein said control unit is adapted to cause the implantable medical device to enter an MRI-safe mode of operation upon reception of a comparator output signal indicating a receiver output signal exceeding said threshold value.

3. The implantable medical device according to claim 1, wherein the magnetic resonance imaging magnetic field detector comprises a giant magnetoresistive ratio sensor that is adapted and arranged to put out a sensor signal that depends on both, magnitude and direction of a magnetic field, and wherein the control unit is adapted to respond to an output signal from said giant magnetoresistive ratio sensor differently depending on whether the sensor output signal represents
a: no or a very low magnetic field up to an order of 0,1 mT or
b: a medium magnetic field in the order of 1 mT or
c: a strong magnetic field in the order of 1 T or more,
the control unit being further adapted to cause the implantable medical device to enter said MRI-safe mode of operation when the sensor output signal represents a strong magnetic field in the order of 1 T or more.

4. The implantable medical device according to claims 2 and 3, wherein the magnetic resonance imaging magnetic field detector comprises both, a giant magnetoresistive ratio sensor according to claim 3 and a bandpass receiver according to claim 2.

5. The implantable medical device according to claim 1, wherein the control unit is adapted to cause the implantable medical device to enter a V00 mode of operation upon reception of a comparator output signal indicating a receiver output signal exceeding said threshold value.

6. The implantable medical device according to claim 1, wherein the control unit is adapted to cause the implantable medical device to enter a D00 mode of operation upon reception of a comparator output signal indicating a receiver output signal exceeding said threshold value.

7. The implantable medical device according to claim 1, wherein the control unit is adapted to cause a system reset of the implantable medical device upon reception of a comparator output signal indicating a receiver output signal exceeding said threshold value.

8. The implantable medical device according to claim 3, wherein the GMR sensor is adapted to recognize a magnetic field vector depending on magnitude and direction of a magnetic field and to compensate said magnetic field vector for B0- and G-fields.

9. The implantable medical device according to claim 2, wherein the antenna of said bandpass receiver is a programming coil of the implantable medical device.
